# DEMANDE DE BREVET EUROPEEN

(11) **EP 1 473 022 A2**
(43) Date de publication de la demande: **03.11.2004**
(21) Numéro de dépôt: 04101246.9
(22) Date de dépôt: 25.03.2004
(51) Int. Cl.: A61K 7/09, A61K 7/135

(54) **Compositions réductrices pour la décoloration ou la déformation permanente de fibres kératiniques comprenant des acides polycarboxyliques et leurs sels comme agents complexants**

(30) Priorité: 25.03.2003 FR 0350066; 28.03.2003 FR 0350079
(71) Demandeur: L'OREAL, 75008 Paris (FR)
(72) Inventeur: Legrand, Frédéric, 92400 Courbevoie (FR)
(74) Mandataire: Poulin, Gérard

(57) **Abrégé**

L'invention se rapporte à des compositions réductrices prêtes à l'emploi pour la décoloration ou la déformation permanente de fibres kératiniques, qui comprennent des acides polycarboxyliques et leur sels en tant qu'agents complexants.

Elle se rapporte également à des procédés et des dispositifs ou "kits" de décoloration ou de déformation permanente de fibres kératiniques, ainsi qu'à l'utilisation de ces compositions, procédés et dispositifs pour la décoloration ou la déformation permanente de fibres kératiniques humaines et, plus spécialement, de cheveux.

## Description

### DOMAINE TECHNIQUE

La présente invention se rapporte à des compositions réductrices pour la décoloration ou la déformation permanente de fibres kératiniques, qui comprennent des acides polycarboxyliques et leur sels en tant qu'agents complexants.

Elle se rapporte également à des procédés et des dispositifs ou "kits" de décoloration ou de déformation permanente de fibres kératiniques, ainsi qu'à l'utilisation de ces compositions, procédés et trousses pour la décoloration ou la déformation permanente de fibres kératiniques humaines et, plus spécialement, de cheveux.

### ETAT DE LA TECHNIQUE ANTERIEURE

Pour décolorer des fibres kératiniques, on utilise deux types de compositions : des compositions dites oxydantes car elles renferment un ou plusieurs agents aptes à oxyder la mélanine des cheveux et, partant, à la solubiliser pour en obtenir l'élimination totale ou partielle, et des compositions dites, au contraire, réductrices car elles contiennent un ou plusieurs agents réducteurs comme l'acide ascorbique, les sulfites et les sulfinates, et qui sont plus spécialement destinées à la décoloration de cheveux ayant été antérieurement teints avec des pigments exogènes.

Par ailleurs, pour la déformation permanente des cheveux, il est usuel d'appliquer sur la chevelure préalablement mise sous tension, par exemple à l'aide de bigoudis si la déformation recherchée est une frisure, une composition contenant un ou plusieurs agents réducteurs de manière à induire l'ouverture des ponts disulfures formés par les résidus cystéine de la kératine des cheveux, puis, généralement après un rinçage, de réoxyder la chevelure pour en fixer la déformation.

Les réducteurs préférentiellement utilisés dans le cadre de la déformation permanente des cheveux sont les thiols tels que l'acide thioglycolique et l'acide thiolactique, leurs sels et leurs esters, et les sulfites.

Qu'elles soient destinées à la décoloration ou à la déformation permanente, les compositions réductrices contiennent en principe un agent destiné à complexer les cations métalliques susceptibles de se trouver à l'état de traces dans ces compositions, ainsi que ceux pouvant être présents sur les cheveux et provenant de l'air ambiant, de l'eau avec laquelle les cheveux ont été lavés ou encore des shampoings ou autres produits capillaires avec lesquels ils ont été traités.

Il est, en effet, très important de neutraliser ces cations métalliques, dans la mesure où ils sont susceptibles de catalyser les réactions de réduction et ce, de façon non contrôlée, ce qui peut se traduire par des effets indésirables sévères tels qu'une cassure des cheveux ou des brûlures du cuir chevelu.

Actuellement, les agents complexants les plus couramment utilisés dans les compositions réductrices pour la décoloration ou la déformation permanente de fibres kératiniques sont l'acide éthylènediamine tétraacétique (EDTA) et ses dérivés comme l'acide diéthylènetriamine pentaacétique (DPTA), généralement dans des proportions pondérales de l'ordre de 0,1 à 1%.

Toutefois, dans le cadre de ses travaux, la Demanderesse a constaté que l'EDTA et ses dérivés présentent, dans ce type de compositions, des propriétés insuffisantes. Ces constatations, qui sont corroborées par les résultats obtenus par d'autres équipes de recherche, justifient de trouver de nouveaux agents complexants.

Un agent complexant destiné à entrer dans la constitution de compositions réductrices pour la décoloration ou la déformation permanente de fibres kératiniques, doit satisfaire à de nombreuses exigences.

En effet, outre qu'il doit présenter un pouvoir complexant élevé vis-à-vis des métaux de manière à supprimer ou, à tout le moins, réduire le plus possible le risque d'une catalyse de la réaction de réduction des fibres kératiniques par les métaux susceptibles d'être présents, même à l'état de traces, dans ces compositions et sur ces fibres, il doit être compatible, et notamment ne pas réagir, avec les autres constituants de ces compositions, et en particulier avec le ou les agents réducteurs.

Il doit également être stable en milieu aqueux, les compositions réductrices étant, en effet, appliquées généralement sur les fibres kératiniques sous forme de compositions aqueuses.

Il doit aussi être d'une innocuité totale pour ces fibres et pour la peau, et être notamment dénué de tout caractère allergène.

Il est, en outre, souhaitable, dans un souci de respect de l'environnement, qu'il soit biodégradable, et que son coût de production ou d'achat autorise son utilisation dans des compositions destinées à être vendues, non seulement à des professionnels, mais également dans la distribution grand public.

Or, après de longues recherches menées sur la question, la Demanderesse a constaté que, de manière surprenante, certains acides polycarboxyliques et leurs sels sont susceptibles de répondre à toutes ces exigences et de représenter, par conséquent, des agents complexants de choix dans des compositions réductrices pour la décoloration et la déformation permanente de fibres kératiniques.

C'est cette constatation qui est à la base de l'invention.

### EXPOSÉ DE L'INVENTION

L'invention a, en premier lieu, pour objet une composition réductrice pour la décoloration ou la déformation permanente de fibres kératiniques, comprenant au moins un agent réducteur, ladite composition étant caractérisée en ce qu'elle comprend au moins un composé répondant à la formule générale (I) suivante :

R-N-(CH(R')CO₂X)₂ (I)

dans laquelle :
- R représente un atome d'hydrogène ou un groupe - CH (CO₂X) - (CH₂)₂CO₂X, -CH₂-CH₂-OH, -CH (CH₃) -CO₂X ou - (CH₂)₂-N (COR") -CH₂-CO₂X ;
- R" représente un groupe alkyle linéaire ou ramifié et comportant de 1 à 30 atomes de carbone, ou bien un groupe alkyle cyclique comportant de 3 à 30 atomes de carbone ;
- R' représente un groupe -CH₂CO₂X lorsque R représente un atome d'hydrogène, tandis que R' représente un atome d'hydrogène lorsque R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium.

Ainsi, les agents complexants utilisés dans le cadre de l'invention, correspondent à des composés acides polycarboxyliques et aux carboxylates correspondants.

Plus précisément, les agents complexants correspondent à :
- des composés comprenant quatre fonctions acides carboxyliques ou carboxylates, lorsque R représente un atome d'hydrogène et R' représente un groupe -CH₂-CO₂X, ou lorsque R représente le groupe - CH (CO₂X) - (CH₂) ₂-CO₂X et R' représente un atome d'hydrogène ;
- des composés comprenant trois fonctions acides carboxyliques ou carboxylates, lorsque R représente le groupe -CH(CH₃)-CO₂X et R' représente un atome d'hydrogène, ou lorsque R représente un groupe - (CH₂)₂-N(COR")-CH₂-CO₂X et R' représente un atome d'hydrogène ; et à
- des composés comprenant deux fonctions acides carboxyliques ou carboxylates, lorsque R représente le groupe -CH₂-CH₂-OH et R' représente un atome d'hydrogène.

Conformément à l'invention, lorsque le ou les composés de formule (I) sont des carboxylates, alors le cation monovalent ou divalent est, de préférence, choisi dans le groupe constitué par les cations de métaux alcalins, les cations de métaux alcalino-terreux, les cations divalents de métaux de transition et les cations monovalents issus d'amines organiques ou d'ammonium.

A titre d'exemples de cations de métaux alcalins, on peut notamment citer le sodium (Na⁺) et le potassium (K⁺), tandis qu'à titre d'exemples de cations de métaux alcalino-terreux, on peut notamment citer le calcium (Ca²⁺) et le magnésium (Mg²⁺) .

Au sens de la présente invention, on entend par "métal de transition", un métal comportant une sous-couche d incomplète, plus particulièrement à l'état d'oxydation II, tel que le cobalt (Co²⁺), le fer (Fe²⁺) , le manganèse (Mn²⁺) , le zinc (Zn²⁺) et le cuivre (Cu²⁺) .

En ce qui concerne les cations d'amines organiques, on peut citer les cations d'amines primaires, secondaires ou tertiaires, ou encore d'alcanolamines.

Lesdites amines présentent un ou plusieurs radicaux, identiques ou non, de type alkyle, linéaire ou ramifié en C₁ à C₂₀, comprenant éventuellement un hétéroatome comme l'oxygène.

Pour ce qui a trait aux cations d'ammonium quaternaires, ces derniers comprenant trois radicaux, identiques ou non, choisis parmi l'hydrogène, un radical alkyle, linéaire ou ramifié en C₁ à C₂₀, comprenant éventuellement un hétéroatome comme l'oxygène.

Conformément à l'invention, lorsque le ou les composés de formule (I) sont des carboxylates, alors le cation monovalent ou divalent est, de préférence, choisi dans le groupe constitué par les cations de métaux alcalins, les cations de métaux alcalino-terreux, les cations divalents de métaux de transition, et les cations issus d'amines organiques ou d'ammonium.

Le ou les composés de formule (I) sont, de préférence, choisis dans le groupe constitué par l'acide méthylglycine diacétique, l'acide 2-hydroxyéthyliminodiacétique, l'acide N-lauroyl-N,N',N'-tri-acétique éthylènediamine, l'acide iminodisuccinique, l'acide N,N-dicarboxyméthyl L-glutamique, leurs sels de métaux alcalins, leurs sels de métaux alcalino-terreux, leurs sels de métaux de transition, leurs sels d'amines organiques, leurs sels d'ammonium, et leurs mélanges.

L'acide méthylglycine diacétique, l'acide 2-hydroxyéthyliminodiacétique, l'acide N-lauroyl-N,N',N'-triacétique éthylènediamine, l'acide iminodisuccinique, l'acide N,N-dicarboxyméthyl L-glutamique et leurs sels sont respectivement représentés par les formules (II), (III), (IV), (V) et (VI) suivantes : 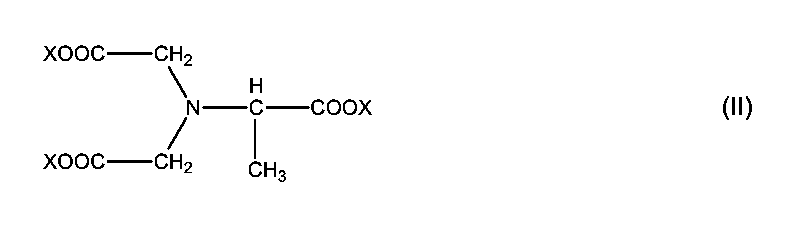 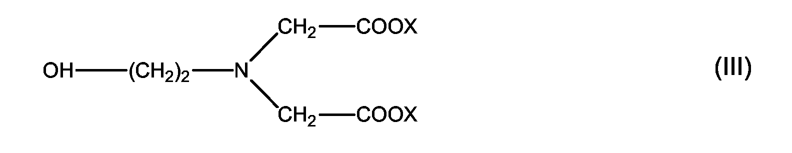 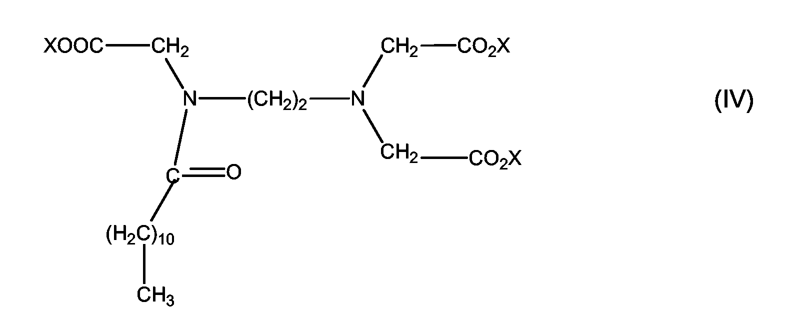 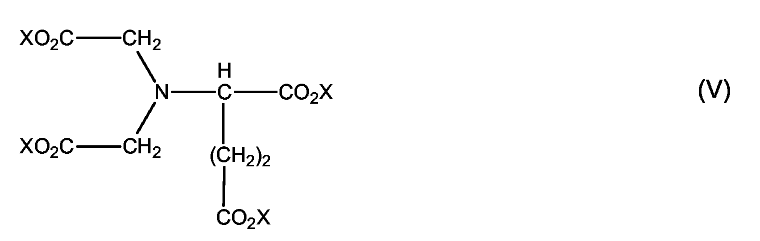 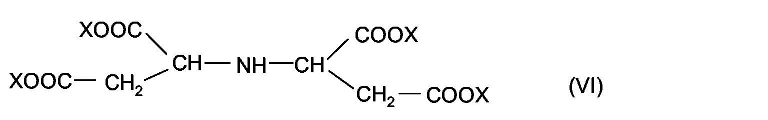 dans lesquelles X est tel que défini précédemment, X correspondant, de préférence, à H ou Na.

Ces composés sont notamment disponibles auprès des sociétés BASF, Dow Chemical, Hampshire, Bayer et Showa Denko.

Plus particulièrement, on préfère l'acide 2-hydroxyéthyliminodiacétique, l'acide méthylglycinediacétique, leurs sels de sodium et leurs mélanges.

De préférence, le ou les composés de formule (I) représentent de 0,001 à 10% en poids et, mieux encore, de 0,001 à 5% en poids du poids total de la composition réductrice. Les pourcentages en poids sont exprimés par rapport à la forme acide du ou des composés de formule (I).

Conformément à l'invention, le ou les agents réducteurs présents dans la composition réductrice peuvent être indifféremment choisis parmi tous les agents réducteurs dont l'utilisation a déjà été proposée dans le domaine de la décoloration et la déformation permanente de fibres kératiniques.

Toutefois, dans le cas d'une composition destinée à la décoloration, ce ou ces agents réducteurs sont, de préférence, choisis dans le groupe constitué par les réductones telles que l'acide ascorbique, l'acide érythorbique, leurs sels et leurs esters, les sulfites comme le sulfite de sodium et les sulfinates comme l'hydroxyméthane-sulfinate de sodium, tandis que, dans le cas d'une composition destinée à la déformation permanente, on préfère utiliser un ou plusieurs thiols, et en particulier l'acide thioglycolique, l'acide thiolactique, la cystéamine, la cystéine, leurs sels et leurs esters, et/ou un ou plusieurs sulfites ou sulfinates.

Il est rappelé que les réductones sont des lactones à caractère réducteur.

Dans tous les cas, le ou les agents réducteurs représentent, préférentiellement, de 0,1 à 30% en poids et, mieux encore, de 0,5 à 20% en poids du poids total de la composition réductrice.

De préférence, la composition réductrice comprend, en plus du ou des composés de formule (I) et du ou des agents réducteurs, un ou plusieurs constituants choisis parmi : (A) les polymères conditionneurs cationiques ou amphotères, (B) les polymères amphiphiles non ioniques, anioniques, cationiques ou amphotères, comportant une chaîne hydrophobe, (C) les agents tensioactifs, (D) les agents d'ajustement de la rhéologie différents des polymères (B), (E) les agents d'ajustement du pH, et/ou (F) les solvants.

### (A) les polymères conditionneurs cationiques ou amphotères :

Au sens de la présente invention, on entend par "polymère conditionneur cationique", tout polymère qui comprend des groupes cationiques ou des groupes ionisables en groupes cationiques et qui permet d'améliorer les propriétés cosmétiques des fibres kératiniques, en particulier le démêlage, la douceur, la brillance, le volume.

Les polymères conditionneurs cationiques ou amphotères convenables sont de manière avantageuse, choisis parmi ceux déjà connus en soi comme améliorant les propriétés cosmétiques des cheveux, à savoir notamment ceux décrits dans les brevets et demandes de brevets EP 337 354, FR 2 270 846, FR 2 383 660, FR 2 598 611, FR 2 470 596, FR 2 519 863, FR 2 788 974, FR 2 788 976.

Cependant, à titre d'exemples plus précis de polymères conditionneurs cationiques, on peut notamment citer les polymères cationiques comprenant au moins des groupements amine primaire, secondaire, tertiaire et/ou quaternaire pouvant, soit faire partie de la chaîne principale polymère, soit être portés par un substituant latéral directement relié à celle-ci.
Ainsi, on peut citer :
(1) les copolymères d'acrylamide et de diméthyl-amino-éthyl méthacrylate quaternisé au sulfate de diméthyle ou avec un halogénure de diméthyle (Hercofloc de Hercules) ; les copolymères d'acrylamide et de chlorure de méthacryloyloxy-éthyl-triméthylammonium (Bina Quat P 100 de Ciba Geigy) ; le copolymère d'acrylamide et de méthosulfate de méthacryloyloxy-éthyl-triméthylammonium (Reten de Hercules) ; les copolymères vinylpyrrolidone/acrylate ou méthacrylate de dialkylaminoalkyle quaternisés ou non (gamme Gafquat d'ISP ; Copolymer 845, 958 et 937 d'ISP) ; les terpolymères méthacrylate de diméthyl amino éthyle/vinylcaprolactame/vinylpyrrolidone (Gaffix VC 713 d'ISP) ; les copolymères vinylpyrrolidone/méthacrylamidopropyl dimethylamine (Styleze CC 10 d'ISP) ; les copolymères vinylpyrrolidone/méthacrylamide de diméthyl-aminopropyle quaternisés (Gafquat HS 100 d'ISP) ;
(2) les dérivés d'éthers de cellulose comportant des groupements ammonium quaternaire tels que décrits dans FR 1 492 597. Ces polymères sont également définis dans le dictionnaire CTFA comme des ammonium quaternaires d'hydroxyéthylcellulose ayant réagi avec un époxyde substitué par un groupement triméthylammonium ;
(3) les dérivés de cellulose cationiques tels que les copolymères de cellulose ou les dérivés de cellulose greffés avec un monomère hydrosoluble d'ammonium quaternaire, et décrits notamment dans US 4,131,576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylamido propyl-triméthylammonium, de diméthyldiallylammonium ;
(4) les polysaccharides cationiques décrits plus particulièrement dans les brevets US 3,589,578, US 4,031,307, tels que les gommes de guar contenant des groupements cationiques trialkylammonium. On utilise par exemple des gommes de guar modifiées par un sel, comme le chlorure notamment, de 2,3-époxypropyl triméthylammonium ;
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans FR 2 162 025, FR 2 280 361 ;
(6) les polyaminoamides solubles dans l'eau préparés en particulier par polycondensation d'un composé acide avec une polyamine, éventuellement réticulés, éventuellement alcoylés ou s'ils comportent une ou plusieurs fonctions amines tertiaires, quaternisées. Ces polymères sont notamment décrits dans FR 2 252 840 et FR 2 368 508 ;
(7) les dérivés de polyaminoamides résultant de la condensation de polyalkylènes polyamines avec des acides polycarboxyliques suivie d'une alcoylation par des agents bifonctionnels. On peut citer par exemple les polymères acide adipiquedialkylamino hydroxyalkyl dialkylène triamine dans lesquels le radical alkyle est en C₁-C₄. De tels polymères sont notamment décrits dans FR 1 583 363 ;
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés en C₃-C₈, puis avec l'épichlorhydrine. De tels polymères sont notamment décrits dans US 3,227,615, US 2,961,347 ;
(9) les cyclopolymères d'alkyl diallyl amine ou de dialkyl diallyl ammonium, sous forme d'homopolymères ou de copolymères, tels que décrits dans FR 2 080 759 et dans son certificat d'addition n°2 190 406 ;
(10) les polymères de diammonium quaternaire tels que décrits dans FR 2 320 330, FR 2 270 846, FR 2 316 271, FR 2 336 434, FR 2 413 907, US 2,273,780, US 2,375,853, US 2,388,614, US 2,454,547, US 3,206,462, US 2,261,002, US 2,271,378, US 3,874,870, US 4,001,432, US 3,929,990, US 3,966,904, US 4,005,193, US 4,025,617, US 4,025,627, US 4,025,653, US 4,026,945 et US 4,027,020; par exemple, on peut citer ceux comprenant les motifs récurrents suivants: 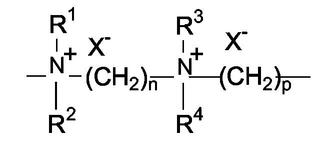 dans laquelle les radicaux R¹, R², R³ et R⁴, identiques ou différents, désignent un radical alkyle ou hydroxyalkyle en C₁-C₄, n et p sont des nombres entiers variant de 2 à 20 et, X⁻ est un anion dérivé d'un acide minéral ou organique ;
(11) les polymères de poly(ammonium quaternaire) constitués de motifs récurrents de formule : 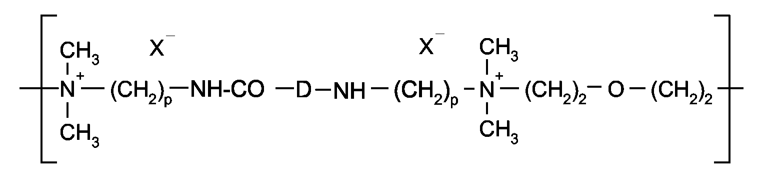 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion. De tels polymères peuvent être préparés selon les procédés décrits dans US 4 157 388, US 4,702,906, US 4,719,282, EP 122 324 ;
(12) les polymères quaternaires de vinylpyrrolidone et de vinylimidazole ;
(13) les polyamines du type polyéthylèneglycol (15) Tallow Polyamine (dénomination du dictionnaire CTFA) ;
(14) les polymères réticulés de sels de méthacryloyloxyalkyl (C₁-C₄) trialkyl (C₁-C₄) ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50% en poids dudit copolymère dans de l'huile minérale (Salcare® SC 92 de Ciba). On peut également utiliser un homopolymère réticulé du chlorure de méthacryloyl oxyéthyl triméthylammonium contenant environ 50% en poids de l'homopolymère dans de l'huile minérale ou dans un ester liquide (Salcare® SC 95, SC 96 de Ciba).

D'autres polymères conditionneurs cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Le ou les polymères conditionneurs amphotères susceptibles d'être présents dans la composition réductrice peuvent, eux, notamment être choisis parmi : ceux comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques ; ou bien K et M peuvent désigner des groupements dérivant de monomères zwittérioniques de carboxybétaïnes ou de sulfobétaïnes ; ou encore K et M désignent une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ; ou bien K et M font partie d'une chaîne d'un polymère à motif éthylène α,β-dicarboxylique dont l'un des groupements carboxyliques a réagi avec une polyamine comportant un ou plusieurs groupements amine primaire ou secondaire.

Les polymères conditionneurs amphotères répondant à la définition donnée ci-dessus plus particulièrement préférés, sont choisis parmi les polymères suivants :
(1) les polymères résultant de la copolymérisation d'un monomère dérivé d'un composé vinylique portant un groupement carboxylique tel que plus particulièrement l'acide (méth)acrylique, l'acide maléique, l'acide alpha-chloracrylique, ou encore un sel de dialkyldiallylammonium tel que le chlorure de diméthyldiallylammonium, et d'un monomère basique dérivé d'un composé vinylique substitué contenant au moins un atome basique tel que plus particulièrement les dialkyl-amino-alkyl-méthacrylate et acrylate, les dialkyl-amino-alkyl-méthacrylamide et acrylamide, comme décrits dans US 3,836,537. On peut citer le copolymère acrylate de sodium/chlorure d'acrylamido propyl triméthyl ammonium (Polyquart KE 3033 de Cognis), le copolymère acide acrylique/ chlorure de diméthyldiallyl ammonium (Merquat 280, 295, Plus 3330, de Nalco) ;
(2) les polymères comportant des motifs dérivant : a) d'au moins un monomère choisi parmi les (méth)acrylamides N-substitués par un radical alkyle, notamment en C₂-C₁₂, b) d'au moins un monomère acide contenant un ou plusieurs groupements carboxyliques réactifs (par exemple acides (méth)acrylique, crotonique, itaconique, et les monoesters des acides ou anhydrides maléique, fumarique), et c) d'au moins un monomère basique tel que des esters à substituant amine primaire, secondaire, tertiaire et quaternaire des acides (méth)acrylique, fumarique, maléique, et le produit de quaternisation du méthacrylate de diméthylamino-éthyle avec le sulfate de diméthyle ou diéthyle. On utilise particulièrement les copolymères octylacrylamide/acrylate/butylaminoéthyl méthacrylate (Amphomer ou Lovocryl 47 par la société National Starch) ;
(3) les polyaminoamides réticulés et partiellement ou totalement alcoylés, dérivant de polyaminoamides de formule générale -[CO-R⁵-CO-Z]- dans laquelle R⁵ est un radical divalent dérivé d'un acide dicarboxylique saturé ou non (par exemple les acides adipique, triméthyl-2,2,4-adipique et triméthyl-2,4,4-adipique, téréphtalique, itaconique), d'un acide monocarboxylique insaturé (comme l'acide (méth) acrylique) , d'un ester d'alcool en C₁-C₆ des acides précités ou d'un radical dérivant de l'addition de l'un de ces acides avec une amine bis-primaire ou secondaire, et Z désigne un radical d'une polyalkylènepolyamine bis-primaire, mono- ou bis-secondaire. De préférence, Z représente entre 60 et 100 moles %, le radical -NH-[(CH₂)ₓ-NH]ₚ- avec x=2 et p=2 ou 3, ou x=3 et p=2 ; ce radical dérivant de la diéthylène triamine, de la triéthylène tétraamine ou de la dipropylène triamine; entre 0 et 40 moles % le radical ci-dessus, dans lequel x=2 et p=1 et qui dérive de l'éthylènediamine, ou le radical dérivant de la pipérazine -N [CH₂CH_{2] 2}N- ; entre 0 et 20 moles %, le radical -NH-(CH₂) ₆-NH- dérivant de l'hexaméthylène diamine. L'agent réticulant de ces polymères est un agent bifonctionnel choisi parmi les épihalohydrines, les diépoxydes, les dianhydrides, les dérivés bis insaturés, et alcoylés par action d'acide acrylique, d'acide chloracétique ou d'une alcane sultone ou de leurs sels de métaux alcalins ;
(4) les polymères comportant au moins des motifs zwittérioniques, comme par exemple le copolymère de méthacrylate de butyle/méthacrylate de diméthyl carboxy-méthyl-ammonio-éthyle (Diaformer Z301, Sandoz) ;
(5) les polymères dérivés du chitosane comportant des motifs monomères répondant aux formules (I), (II), (III) suivantes : 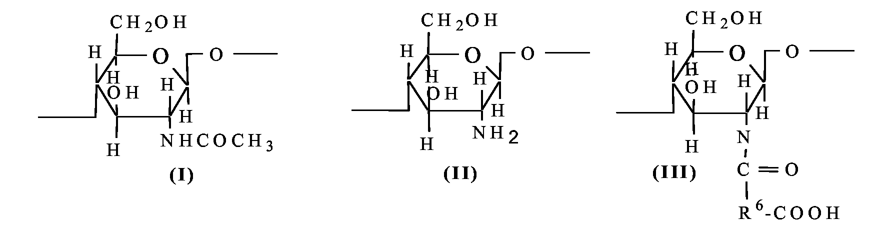 avec (I) représentant de 0 à 30%, (II) de 5 à 50% et (III) de 30 à 90% dans lequel R⁶ représente un radical de formule : 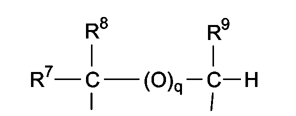 dans laquelle q désigne zéro ou 1 ; et si q=0, les R⁷, R⁸, R⁹, identiques ou différents, représentent un hydrogène, un groupement méthyle, hydroxyle, acétoxy, amino, mono- ou di-alkylamine éventuellement interrompus par un ou plusieurs atomes d'azote et/ou éventuellement substitués par un ou plusieurs groupes amine, hydroxyle, carboxyle, alkylthio éventuellement porteur d'un groupe amino, sulfonique ; ou si q=1, les R⁷, R⁸, R⁹, identiques ou différents, représentent un hydrogène, ainsi que les sels formés par ces composés avec des bases ou des acides ;
(6) les polymères dérivés de la N-carboxyalkylation du chitosane comme le N-carboxyméthyl chitosane ou le N-carboxybutyl chitosane (Evalsan de Jan Dekker) ;
(7) les polymères tels que décrits dans FR 1 400 366 : 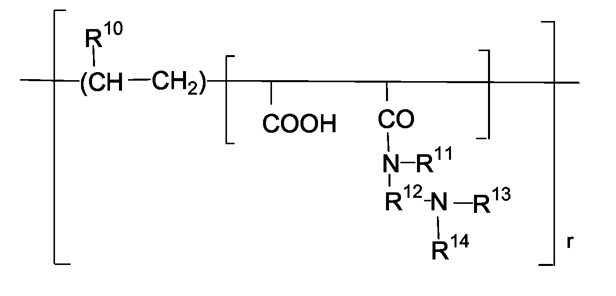 dans laquelle R¹⁰ est un hydrogène, CH₃O-, CH₃CH₂O-, phényle, R¹¹ et R¹⁴, identiques ou différents, représentent un hydrogène, un radical alkyle (méthyle, éthyle), R¹³ représente un radical alkyle (méthyle, éthyle) ou un radical de formule -R¹²-N (R¹⁴)₂, R¹² représentant - (CH₂)₂-, - (CH₂)₃-, -CH₂-CH (CH₃) -, ainsi que les homologues supérieurs de ces radicaux et contenant jusqu'à 6 atomes de carbone, r est tel que le poids moléculaire est compris entre 500 et 6000000 et de préférence entre 1000 et 1000000 ;
(8) les polymères amphotères du type - D¹-X-D¹-X- choisis parmi:
   a) les polymères obtenus par action de l'acide chloracétique ou le chloracétate de sodium sur les composés comportant au moins un motif de formule - D¹-X-D¹-X-D¹- où D¹ désigne un radical pipérazinyle et X désigne le symbole E ou E', E ou E' identiques ou différents désignent un radical bivalent qui est un radical alkylène à chaîne droite ou ramifiée comportant jusqu'à 7 atomes de carbone dans la chaîne principale substituée ou non par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule -D¹-X-D¹-X- où D¹ désigne un radical pipérazinyle et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude ;
(9) les copolymères alkyl(C₁-C₅)-vinyléther/anhydride maléique modifié partiellement par semi-amidification avec une N,N-dialkylaminoalkylamine telle que la N,N-diméthyl-amino-propylamine, ou par semi-estérification avec une N,N-dialcanolamine. Ces copolymères peuvent également comporter d'autres comonomères vinyliques tels que le vinylcaprolactame.

Parmi les polymères conditionneurs ou amphotères utilisables, on préfère notamment :
(i) parmi les cationiques :
   - l'homopolymère de chlorure de diméthyldiallylammonium (Merquat 100 de Nalco);
   - les copolymères de chlorure de diméthyldiallylammonium et d'acrylamide (Merquat 2200 de Nalco);
   - les polymères de type poly(ammonium quaternaire) préparés et décrits dans FR 2 270 846, constitués de motifs récurrents de formules (W) et (U) suivantes : 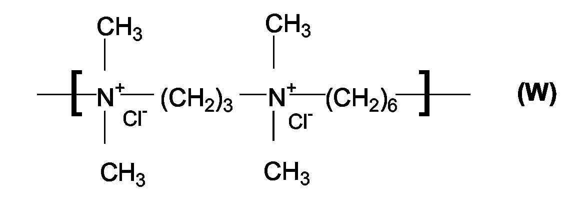 et notamment ceux dont le poids moléculaire, déterminé par chromatographie par perméation de gel, est compris entre 9500 et 9900 ; 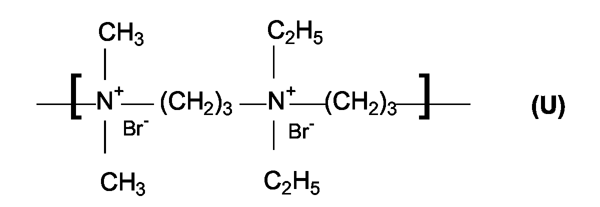 et notamment ceux dont le poids moléculaire, déterminé par chromatographie par perméation de gel, est d'environ 1200 ;
   - les polymères de type poly(ammonium quaternaire) de la famille (11) avec X⁻ désignant le chlore, et notamment ceux dont la masse moléculaire moyenne en poids est inférieure à 100 000, de préférence inférieure ou égale à 50 000;
(ii) parmi les polymères amphotères :
   - le copolymère chlorure de diméthyldiallylammonium/acide acrylique (80/20) (Merquat 280 de Nalco- dénomination CTFA : Polyquaternium 22) ;
   - le copolymère chlorure de diméthyldiallylammonium/acide acrylique (95/5) (Merquat 295 de Nalco) ;
   - le copolymère de chlorure de méthacrylamidopropyl trimonium, d'acide acrylique et d'acrylate d'éthyle (Merquat 2001 de Nalco-dénomination CTFA : Polyquaternium 47) ;
   - le terpolymère acrylamide/chlorure de diméthyldiallyl ammonium/acide acrylique (Merquat Plus 3330 de Nalco -dénomination CTFA : Polyquaternium 39).

Lorsque la composition réductrice comprend un ou plusieurs polymères conditionneurs cationiques ou amphotères, alors celui-ci ou ceux-ci représentent généralement de 0,01 à 10% en poids et, mieux encore, de 0,05 à 5% du poids total de cette composition.

### (B) les polymères amphiphiles non ioniques, anioniques, cationiques ou amphotères, comportant une chaîne hydrophobe :

Plus particulièrement, la chaîne hydrophobe est une chaîne hydrocarbonée, saturée ou non, aromatique ou non, linéaire ou ramifiée, en C₆-C₃₀, comprenant éventuellement un ou plusieurs motifs oxyalkylénés (oxyéthylénés et/ou oxypropylénés).

Parmi les polymères amphiphiles cationiques comportant une chaîne hydrophobe, on peut trouver des polyuréthannes cationiques ou des copolymères cationiques comprenant des motifs vinyllactame et en particulier vinylpyrrolidone.

De préférence, les polymères amphiphiles comportant une chaîne hydrophobe sont de nature non ionique ou anionique.

A titre d'exemples de polymères amphiphiles non ioniques à chaîne hydrophobe, on peut citer entre autres :
(1) les celluloses modifiées par des groupements comportant au moins une chaîne hydrocarbonée, saturée ou non, linéaire ou ramifiée, en C₆-C₃₀, comme les hydroxyéthylcelluloses modifiées par des groupements comportant au moins une chaîne hydrophobe telle que définie auparavant, comme notamment Natrosol Plus Grade 330 CS (alkyles en C₁₆ - commercialisé par la société Aqualon) ; Bermocoll EHM 100 (commercialisé par la société Berol Nobel), Amercell Polymer HM-1500 (hydroxyéthylcellulose modifiée par un groupement polyéthylène glycol (15) éther de nonylphénol - commercialisé par la société Amerchol) ;
(2) les hydroxypropylguars modifiés par des groupements comportant au moins une chaîne hydrophobe telle que définie, par exemple Jaguar XC-95/3 (chaîne alkyle en C₁₄ - commercialisé par la société Rhodia Chimie) ; Esaflor HM 22 (chaîne alkyle en C₂₂ - commercialisé par la société Lamberti) ; RE210-18 (chaîne alkyle en C₁₄) et RE205-1 (chaîne alkyle en C₂₀) commercialisés par la société Rhodia Chimie ;
(3) les copolymères de vinylpyrrolidone et de monomères hydrophobes à chaîne hydrophobe telle que définie auparavant comme par exemple Antaron ou Ganex V216 (copolymères vinylpyrrolidone/hexadécène) ; Antaron ou Ganex V220 (copolymères vinylpyrrolidone/eicosène), commercialisés par la société I.S.P ;
(4) les copolymères de (méth)acrylates d'alkyles en C₁-C₆ et de monomères amphiphiles comportant une chaîne hydrophobe ;
(5) les copolymères de (méth)acrylates hydrophiles et de monomères hydrophobes comportant au moins une chaîne hydrophobe, tels que par exemple le copolymère méthacrylate de polyéthylèneglycol/méthacrylate de lauryle ;
(6) les polymères à squelette aminoplaste éther possédant au moins une chaîne grasse, tels que les composés Pure Thix commercialisés par la société Süd-Chemie ;
(7) les polyéthers polyuréthannes, linéaires (structure à blocs), greffés ou en étoile, comportant dans leur chaîne, au moins une séquence hydrophile, généralement polyoxyéthylénée et pouvant comprendre entre 50 et 1000 motifs oxyéthylène environ, et au moins une séquence hydrophobe, qui peut comprendre des groupements aliphatiques seuls, éventuellement combinés à des enchaînements cycloaliphatiques et/ou aromatiques. De préférence, les polyéthers polyuréthannes comportent au moins deux chaînes hydrophobes hydrocarbonées en C₆-C₃₀, séparées par une séquence hydrophile ; les chaînes hydrophobes pouvant être des chaînes pendantes ou des chaînes à l'une ou plusieurs des extrémités de la ou des séquences hydrophiles.

Les polyéthers polyuréthannes comportent une liaison uréthanne entre les séquences hydrophiles, mais peuvent aussi comprendre des séquences hydrophiles liées aux séquences lipophiles par d'autres liaisons chimiques.

Les polyéthers polyuréthannes sont en particulier ceux décrits dans l'article de G. Fonnum, J. Bakke et Fk. Hansen - Colloid Polym. Sci.271, 380-389 (1993). A titre d'exemples de polyéthers polyuréthannes, on peut citer Nuvis FX 1100 (désignation I.N.C.I. européenne et américaine "Steareth-100/PEG-136/H.M.D.I. Copolymer" commercialisé par la société Servo Delden) ; Rheolate 205, 208, 204 ou 212 (commercialisés par la société Rheox) ; Elfacos T210 (chaîne alkyle en C₁₂- C₁₄) Elfacos T212 (chaîne alkyle en C₁₈) commercialisés par la société Akzo.

Les polymères amphiphiles anioniques à chaîne hydrophobe, susceptibles d'être mis en oeuvre comportent au moins à titre de chaîne hydrophobe, une chaîne hydrocarbonée, saturée ou non, aromatique ou non, linéaire ou ramifiée, en C₈-C₃₀.

Plus particulièrement les polymères amphiphiles anioniques comportant au moins une chaîne hydrophobe, réticulés ou non, comprennent au moins un motif hydrophile dérivé d'un ou de plusieurs monomères à insaturation éthylénique portant une fonction acide carboxylique, ou une fonction acide sulfonique, libre ou partiellement ou totalement neutralisée, et au moins un motif hydrophobe dérivé d'un ou de plusieurs monomères à insaturation éthylénique portant une chaîne latérale hydrophobe, et éventuellement au moins un motif de réticulation dérivés d'un ou plusieurs monomères polyinsaturés.

Des polymères amphiphiles anioniques du type décrit ci-dessus sont décrits et préparés, par exemple dans les brevets US 3,915,921 et US 4,509,949 (copolymères d'acide (méth)acrylique et de (méth)acrylates d'alkyles en C₁₀-C₃₀) ou dans le brevet EP 216 479 (copolymères d'acide (méth)acrylique et d'éthers allyliques d'alcools gras).

Les polymères amphiphiles comportant au moins un groupement sulfonique, sous forme libre ou partiellement ou totalement neutralisée et au moins une partie hydrophobe sont par exemple décrits dans FR 00 16954 et FR 01 00328 dont le contenu fait partie intégrante de la présente invention.

On peut citer plus particulièrement parmi eux le copolymère acide acrylamido-2-méthyl-2-propane-sulfonique (AMPS)/n-dodécylacrylamide neutralisé par la soude, le copolymère réticulé par du méthylène-bis-acrylamide constitué de 75% en poids de motifs AMPS neutralisés par NH₃ et de 25% en poids de motifs acrylate de Genapol T-250, le copolymère réticulé par du méthacrylate d'allyle constitué de 90% en poids de motifs AMPS neutralisés par NH₃ et de 10% en poids de motifs méthacrylate de Genapol T-250, ou le copolymère réticulé par du méthacrylate d'allyle constitué de 80% en poids de motifs AMPS neutralisés par NH₃ et de 20% en poids de motifs méthacrylate de Genapol T-250.

On peut citer à titre d'exemples de polymères préférés, Carbopol ETD-2020 (copolymère acide acrylique/méthacrylate d'alkyle en C₁₀-C₃₀, réticulé - commercialisé par la société Noveon) ; Carbopol 1382, Pemulen TR1, Pemulen TR2 (copolymères acide acrylique/acrylate d'alkyle en C₁₀-C₃₀, réticulés - commercialisés par la société Noveon) le copolymère acide méthacrylique/acrylate d'éthyle/méthacrylate de stéaryle oxyéthyléné (55/35/10); le copolymère acide (méth)acrylique/acrylate d'éthyle/méthacrylate de béhényle oxyéthyléné 25 OE (Aculyn 28 commercialisé par Rohm & Haas) et le copolymère réticulé acide méthacrylique/acrylate d'éthyle/stéareth-10 allyl éther.

Lorsque la composition réductrice comprend un ou polymères amphiphiles à chaîne grasse, alors celui-ci ou ceux-ci représentent généralement de 0,05 à 20% en poids et, mieux encore, de 0,1 à 10% du poids total de cette composition.

### (C) les agents tensioactifs :

Le ou les agents tensioactifs susceptibles d'être présents dans la composition réductrice peuvent être indifféremment choisis parmi les tensioactifs anioniques, non ioniques, amphotères et cationiques.

Des agents tensioactifs anioniques, non ioniques, amphotères ou cationiques convenant à la mise en oeuvre de l'invention sont notamment les suivants :

### • tensioactifs anioniques :

A titre d'exemples d'agents tensioactifs anioniques susceptibles d'être utilisés, seuls ou en mélanges, on peut citer les sels, en particulier les sels alcalins (sels de sodium, sels de magnésium, sels d'ammonium, sels d'amines, sels d'aminoalcools, ...) des composés suivants : alkylsulfates, alkyléthersulfates, alkylamidoéthersulfates, alkylarylpolyéthersulfates, monoglycérides sulfates ; alkylsulfonates, alkylphosphates, alkylamidesulfonates, alkylarylsulfonates, α-oléfine-sulfonates, paraffine-sulfonates ; alkyl(C₆-C₂₄) sulfosuccinates, alkyl (C₆-C₂₄) éthersulfosuccinates, alkyl (C₆-C₂₄) amidesulfosuccinates, alkyl (C₆-C₂₄) sulfoacétates ; acyl (C₆-C₂₄) sarcosinates et acyl (C₆-C₂₄)glutamates.

On peut aussi citer les esters d'alkyl(C₆-C₂₄)polyglycosides carboxyliques tels que les alkylpolyglucoside citrates, les alkylpolyglucoside tartrates, les alkylpolyglucoside sulfosuccinates et les alkylpolyglucoside sulfosuccinamates ; les acyliséthionates et les N-acyltaurates, le radical alkyle ou acyle de tous ces composés comportant, de préférence, de 12 à 20 atomes de carbone, et le radical aryle désignant, de préférence, un groupement phényle ou benzyle.

Sont également utilisables les sels d'acides gras tels que les sels des acides oléique, ricinoléique, palmitique et stéarique, des acides d'huile de coprah ou d'huile de coprah hydrogénée ; les acyllactylates dont le radical acyle comporte de 8 à 20 atomes de carbone ; les acides d'alkyl D galactoside uroniques et leurs sels ; les acides alkyl (C₆-C₂₄) éther carboxyliques polyoxyalkylénés, les acides alkyl(C₆-C₂₄)amido éther carboxyliques polyoxyalkylénés et leurs sels, en particulier ceux comportant de 2 à 50 groupes oxydes d'alkylène et, plus spécialement oxydes d'éthylène, et leurs mélanges.

### • tensioactifs non ioniques :

Les agents tensioactifs non ioniques sont des composés bien connus en eux-mêmes (voir, par exemple, le "Handbook of Surfactants", M.R. PORTER, Ed. Blackie & Son, Glasgow and London, 1991, 116-178) et leur nature ne revêt pas, dans le cadre de la présente invention, de caractère critique.

Ainsi, utilisés seuls ou en mélanges, ils peuvent notamment être choisis parmi les alcools, les α-diols, les alkylphénols polyéthoxylés et polypropoxylés ayant une chaîne grasse comportant, par exemple, de 8 à 18 atomes de carbone, le nombre de groupes oxydes d'éthylène ou oxydes de propylène pouvant être notamment de 2 à 50 ; les copolymères d'oxyde d'éthylène et de propylène, les condensats d'oxyde d'éthylène et de propylène sur des alcools gras, les amides gras polyéthoxylés ayant, de préférence, de 2 à 30 moles d'oxyde d'éthylène ; les amides gras polyglycérolés comportant en moyenne de 1 à 5 et, plus spécialement, de 1,5 à 4, groupes glycérol ; les esters d'acide gras du sorbitan oxyéthylénés comportant de 2 à 30 moles d'oxyde d'éthylène ; les esters d'acides gras du sucrose, les esters d'acides gras du polyéthylèneglycol ; les alkylpolyglycosides ; les dérivés de N-alkyl glucamine et les oxydes d'amines tels que les oxydes d'alkyl (C₁₀-C₁₄) amines ou les oxydes de N-acylaminopropylmorpholine.

### • tensioactifs amphotères :

Les agents tensioactifs amphotères (ou zwittérioniques), dont la nature ne revêt pas de caractère critique dans le cadre de la présente invention, peuvent notamment être choisis, seuls ou en mélanges, parmi les dérivés d'amines secondaires ou tertiaires aliphatiques dont le radical aliphatique est une chaîne linéaire ou ramifiée comportant de 8 à 18 atomes de carbone et contenant au moins un groupe anionique hydrosolubilisant, par exemple un carboxylate, un sulfonate, un sulfate, un phosphate ou un phosphonate.

On peut également citer les alkyl (C₈-C₂₀) bétaïnes, les sulfobétaïnes, les alkyl(C₈-C₂₀)amidoalkyl (C₁-C₆) bétaïnes et les alkyl (C₈-C₂₀) amidoalkyl (C₁-C₆)sulfobétaïnes.

Parmi les dérivés d'amines, on peut notamment citer les composés commercialisés par la société Rhodia Chimie sous la dénomination commerciale Miranol® , qui sont décrits dans US 2,528,378 et US 2,781,354 et qui sont classés dans le Dictionnaire CTFA, 5^{ème} édition, 1993, sous les désignations anglo-saxonnes "disodium cocoamphodiacetate", "disodium lauroamphodiacetate", "disodium caprylamphodiacetate", "disodium capryloamphodiacetate", "disodium cocoamphodipropionate", "disodium lauroamphodipropionate", "disodium caprylamphodipropionate", "disodium capryloamphodipropionate", "lauroamphodiproponic acid" et "cocoamphodipropionic acid".

### • tensioactifs cationiques :

Comme agents tensioactifs cationiques aptes à être utilisés seuls ou en mélanges, on peut citer les sels d'amines grasses primaires, secondaires et tertiaires, éventuellement polyoxyalkylénées ; les sels d'ammonium quaternaire tels que les chlorures et les bromures de tétraalkylammonium, d'alkylamidoalkyltrialkylammonium, de trialkylbenzylammonium, de trialkylhydroxyalkylammonium et d'alkylpyridinium ; les dérivés d'imidazoline et les oxydes d'amines à caractère cationique.

Lorsque la composition réductrice comprend un ou plusieurs agents tensioactifs, alors celui-ci ou ceux-ci représentent généralement de 0,01 à 40% en poids et, mieux encore, de 0,1 à 30% du poids total de cette composition.

### (D) les agents d'ajustement de la rhéologie autres que les polymères (B) :

Au sens de la présente invention, on entend par "agent d'ajustement de la rhéologie", tout composé propre à conférer une viscosité à la composition réductrice telle que, une fois appliquée sur des fibres kératiniques, cette dernière ne coule pas et reste parfaitement localisée au point d'application.

Notons que ledit agent est dépourvu de chaîne hydrophobe, c'est-à-dire de chaîne hydrocarbonée, saturée ou non, aromatique ou non, linéaire ou ramifiée, en C₈-C₃₀, comprenant éventuellement un ou plusieurs motifs oxyalkylénés (oxyéthylénés et/ou oxypropylénés).

Le ou les agents d'ajustement de la rhéologie susceptibles d'être présents dans la composition réductrice sont des polymères d'origine naturelle ou les polymères synthétiques, et sont avantageusement choisis parmi ceux utilisés classiquement dans le domaine cosmétique.

Comme exemples de polymères synthétiques, on peut citer, la polyvinylpyrrolidone, l'acide polyacrylique, le polyacrylamide, l'acide poly-2-acrylamidopropanesulfonique non réticulé (Simugel EG de la société SEPPIC), l'acide poly-2-acrylamido-2-méthylpropane sulfonique réticulé, libre ou partiellement neutralisé par l'ammoniaque (Hostacerin AMPS de Clariant), des mélanges d'acide poly-2-acrylamido-2-méthylpropane sulfonique non réticulé avec des éthers d'hydroxyalkylcellulose ou avec des poly(oxyde d'éthylène) tels que décrits dans le brevet US 4,540,510 ; des mélanges d'acide poly(méth)-acrylamido-alkyl(C₁-C₄)-sulfonique, de préférence réticulé, avec un copolymère réticulé de l'anhydride maléique et d'un alkyl(C₁-C₅)vinyléther (Hostacerin AMPS/Stabileze QM de la société ISF).

Les polymères épaississants d'origine naturelle sont de préférence des polymères comportant au moins un motif sucre, comme les gommes de guar non ioniques, modifiées ou non par des groupements hydroxyalkyle en C₁-C₆ ; les gommes de biopoly saccharides d'origine microbienne telles que les gommes de scléroglucane ou de xanthane ; les gommes issues d'exudats végétaux telles que les gommes arabique, ghatti, karaya, tragacanthe, carraghénanne, agar et caroube ; les pectines ; les alginates ; les amidons ; les hydroxyalkyl(C₁-C₆)celluloses et carboxyalkyl(C₁-C₆)celluloses.

Notons que les termes "motif sucre" désignent une portion monosaccharidique (c'est-à-dire monosaccharide ou oside ou sucre simple), une portion oligosaccharidique (chaînes courtes formées de l'enchaînement d'unités monosaccha ridiques, éventuellement différentes) ou une portion polysaccharidique [longues chaînes constituées d'unités monosaccharidiques, éventuellement différentes, c'est-à-dire polyholosides ou polyosides]. Les unités saccharidiques peuvent être en outre substituées par des radicaux alkyle, ou hydroxyalkyle, ou alcoxy, ou acyloxy, ou carboxyle, les radicaux alkyle en C₁-C₄.

A titre d'exemples de gommes de guar non ioniques non modifiées, on peut citer entre autres Guargel D/15 (Noveon) ; Vidogum GH 175 (Unipectine), Meypro-Guar 50 et Jaguar C (Meyhall/Rhodia Chimie) ; et à titre de gommes de guar non ioniques modifiées, Jaguar HP8, HP60, HP120, DC 293, HP 105 (Meyhall/Rhodia Chimie) ; Galactasol 4H4FD2 (Aqualon).

Les gommes de biopolysaccharides d'origine microbienne, végétale sont bien connues de l'homme de l'art et décrites notamment dans l'ouvrage de Robert L. Davidson intitulé "Handbook of Water soluble gums and resins" édité chez Mc Graw Hill Book Company (1980).

Parmi ces gommes, citons les scléroglucanes comme notamment Actigum CS de Sanofi Bio Industries ; Amigel de Alban Muller International, ainsi que les scléroglucanes traités au glyoxal décrits dans FR 2 633 940) ; les gommes xanthanes comme Keltrol, Keltrol T, Keltrol Tf, Keltrol Bt, Keltrol Rd, Keltrol Cg (Nutrasweet Kelco), Rhodicare S, Rhodicare H (Rhodia Chimie) ; les dérivés d'amidon comme Primogel (Avebe) ; les hydroxyéthylcelluloses telles que Cellosize QP3L, QP4400H, QP30000H, HEC30000A, Polymer PCG10 (Amerchol), Natrosol 250HHR, 250MR, 250M, 250HHXR, 250HHX, 250HR, HX (Hercules), Tylose H1000 (Hoechst) ; les hydroxypropylcelluloses comme Klucel EF, H, LHF, MF, G (Aqualon) ; les carboxyméthylcelluloses comme Blanose 7M8/SF, raffinée 7M, 7LF, 7MF, 9M31F, 12M31XP, 12M31P, 9M31XF, 7H, 7M31, 7H3SXF (Aqualon), Aquasorb A500 (Hercules), Ambergum 1221 (Hercules), Cellogen HP810A, HP6HS9 (Montello), Primellose (Avebe).

La composition peut de plus comprendre, en remplacement ou en association avec au moins un agent d'ajustement de la rhéologie, au moins un alkylamide d'acide carboxylique en C₆-C₃₀, linéaire ou non, saturé ou non, et portant éventuellement un ou plusieurs groupements hydroxyle.

Par ailleurs, l'azote du groupement amide peut être mono- ou disubstitué. Il est de préférence monosubstitué.

L'amide peut comprendre 1 à 20 motifs oxyalkylénés (oxyéthylénés et/ou oxypropylénés), de préférence, oxyéthylénés.

Lorsque la composition réductrice comprend un ou plusieurs agents d'ajustement de la rhéologie, alors celui-ci ou ceux-ci représentent généralement de 0,05 à 20% en poids et, mieux encore, de 0,1 à 10% du poids total de cette composition.

### (E) les agents d'ajustement du pH :

Le pH de la composition réductrice peut être compris entre 1,5 et 12.

Toutefois, on préfère que ce pH soit compris entre 1,5 et 10 et, mieux encore, entre 1,5 et 7 dans le cas où la composition réductrice est destinée à la décoloration de fibres kératiniques, et qu'il soit compris entre 6 et 12 et, de préférence, entre 7 et 11 lorsque ladite composition est destinée à la déformation permanente de fibres kératiniques.

De telles valeurs de pH peuvent être obtenues au moyen d'agents acidifiants ou alcalinisants.

A titre d'exemples d'agents acidifiants susceptibles d'être utilisés, on peut citer les acides minéraux ou organiques comme l'acide chlorhydrique, l'acide phosphorique, l'acide orthophosphorique, l'acide acétique, l'acide tartrique, l'acide citrique, l'acide lactique, l'acide borique et les acides sulfoniques.

En ce qui concerne les agents alcalinisants, il est à noter que la déformation permanente de fibres kératiniques envisagée dans le cadre de l'invention n'est pas une lanthionisation. De ce fait, les agents alcalinisants sont, de préférence, choisis parmi des composés autres que les hydroxydes métalliques et les hydroxydes de guanidine, comme par exemple l'ammoniaque, les carbonates alcalins ou d'ammonium, les alcanolamines telles que les mono-, di- et triéthanolamines ainsi que leurs dérivés, les hydroxyalkylamines, les éthylène-diamines oxyéthylénées et/ou oxypropylénées, et les composés répondant à la formule (XIX) suivante : 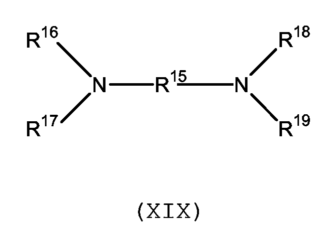 dans laquelle :
- R¹⁵ est un reste propylène éventuellement substitué par un groupement hydroxyle ou un radical alkyle en C₁-C₄ ; tandis que
- R¹⁶, R¹⁷, R¹⁸ et R¹⁹, qui sont identiques ou différents, représentent un atome d'hydrogène, un radical alkyle en C₁-C₄ ou un radical hydroxyalkyle en C₁-C₄.

Lorsque la composition réductrice comprend un ou plusieurs agents acidifiants ou un ou plusieurs agents alcalinisants, alors celui-ci ou ceux-ci représentent généralement de 0,01 à 30% en poids du poids total de cette composition.

### (F) les solvants :

Les solvants susceptibles d'être présents dans la composition réductrice sont notamment l'eau et des mélanges composés d'eau et d'un ou plusieurs solvants organiques acceptables sur le plan cosmétique, ce ou ces solvants organiques pouvant être en particulier des alcools tels que l'éthanol, l'isopropanol, l'alcool benzylique, l'alcool phényléthylique ou l'alcool cétylique, des polyols comme le propylèneglycol et le glycérol ; des éthers de glycols comme les éthers monométhylique, monoéthylique et monobutylique d'éthylèneglycol, ainsi que des alkyléthers de glycols comme le monoéthyléther ou le monobutyléther du diéthylèneglycol.

Ce ou ses solvants organiques, lorsqu'ils sont présents dans la composition réductrice, représentent généralement de 0,5 à 20% en poids et, mieux encore, de 2 à 10% en poids du poids total de cette composition.

La composition réductrice peut encore comprendre, selon l'usage auquel elle est destinée et les propriétés particulières que l'on souhaite lui conférer en fonction de cet usage, un ou plusieurs adjuvants choisis parmi les charges minérales ou organiques telles que la silice ou les argiles, les liants tels que la vinylpyrrolidone, les huiles ou les cires, les polyalkylèneglycols ou les dérivés de polyalkylèneglycols, les lubrifiants tels que les stéarates de polyols ou les stéarates de métaux alcalins ou alcalino-terreux, les agents antimousse, les silicones volatiles ou non volatiles, cycliques, linéaires ou ramifiées, et éventuellement modifiées, notamment par des groupements amines, les agents colorants, les agents matifiants comme les oxydes de titane, les conservateurs, et/ou les parfums.

Chacun de ces adjuvants peut représenter, lorsqu'il est présent dans la composition réductrice, jusqu'à 30% en poids du poids total de cette composition.

La composition réductrice comprend, de préférence, un agent complexant de formule (I) et une réductone et/ou un sulfinate en tant qu'agent(s) réducteur(s) dans le cas où elle est destinée à la décoloration de fibres kératiniques, tandis qu'elle comprend, de préférence, un agent complexant et un thiol et/ou un sulfite et/ou un sulfinate en tant qu'agent(s) réducteur(s), en présence d'un polymère cationique choisi parmi les polymères cationiques précités dans le cas où elle est destinée à la déformation permanente de fibres kératiniques.

L'invention a aussi pour objet un procédé de décoloration ou de déformation permanente de fibres kératiniques, comprenant les étapes consistant à :
a) appliquer sur les fibres kératiniques une composition réductrice tel que précédemment définie ;
b) laisser reposer la composition réductrice sur les fibres kératiniques pendant un temps suffisant pour obtenir la décoloration ou la déformation recherchée ;
c) rincer les fibres kératiniques pour en éliminer la composition réductrice ;
d) laver les fibres kératiniques une ou plusieurs fois, les rincer après chaque lavage et, éventuellement, les sécher ;
ledit procédé comprenant de plus, entre les étapes c) et d), dans le cas d'une déformation permanente, les étapes consistant à : i) appliquer sur les fibres kératiniques une composition oxydante, par exemple à base de peroxyde d'hydrogène ; ii) laisser reposer la composition oxydante sur les fibres kératiniques pendant un temps suffisant pour obtenir la fixation de la déformation recherchée ; et iii) rincer les fibres kératiniques à l'eau pour en éliminer la composition oxydante.

A l'étape b), le temps pendant lequel on laisse la composition réductrice reposer sur les fibres kératiniques peut varier de 1 à 60 minutes, mais est, de préférence, compris entre 10 et 45 minutes, tandis que, à l'étape ii), le temps de repos de la composition oxydante sur les fibres kératiniques est de l'ordre de 1 à 20 minutes et, de préférence, de 1 à 10 minutes.

Dans le cas d'une déformation permanente, des moyens mécaniques de mise sous tension des fibres kératiniques tels que des bigoudis, peuvent être utilisés avant, pendant ou après application de la composition réductrice et être ôtés avant ou après le rinçage de la composition oxydante.

L'invention a aussi pour objet un dispositif ou "kit" pour la décoloration de fibres kératiniques, comprenant au moins deux compositions A et B destinées à être mélangées ensemble pour obtenir une composition réductrice prête à l'emploi, ledit dispositif étant caractérisé en ce que l'une au moins des compositions A et B contient un ou plusieurs agents réducteurs, et l'une au moins des compositions A et B contient un ou plusieurs composés répondant à la formule générale (I) suivante :

R-N-(CH(R')CO₂X)₂ (I)

dans laquelle :
- R représente un atome d'hydrogène ou un groupe -CH(CO₂X)-(CH₂)₂CO₂X, -CH₂-CH₂-OH, -CH(CH₃)-CO₂X ou - (CH₂) ₂-N (COR") -CH₂-CO₂X ;
- R" représente un groupe alkyle linéaire ou ramifié et comportant de 1 à 30 atomes de carbone, ou bien un groupe alkyle cyclique et comportant de 3 à 30 atomes de carbone ;
- R' représente un groupe -CH₂CO₂X lorsque R représente un atome d'hydrogène, tandis que R' représente un atome d'hydrogène lorsque R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium.

L'invention a encore pour objet un dispositif ou "kit" pour la déformation permanente de fibres kératiniques, comprenant, d'une part, soit une composition A, soit au moins deux compositions A' et B' destinées à être mélangées ensemble pour obtenir une composition réductrice prête à l'emploi, et, d'autre part, une composition oxydante C prête à l'emploi ou au moins deux compositions D et E destinées à être mélangées ensemble pour obtenir une composition oxydante prête à l'emploi, ledit dispositif étant caractérisé en ce que, soit la composition A, soit l'une au moins des compositions A' et B' contient un ou plusieurs agents réducteurs, et, soit la composition A, soit l'une au moins des compositions A' et B' contient au moins un ou plusieurs composés répondant à la formule générale (I) suivante :

R-N-(CH(R')CO₂X)₂ (I)

dans laquelle :
- R représente un atome d'hydrogène ou un groupe - CH(CO₂X) - (CH₂)₂CO₂X, -CH₂-CH₂-OH, -CH(CH₃) -CO₂X ou - (CH₂)₂-N(COR") -CH₂-CO₂X ;
- R" représente un groupe alkyle, linéaire ou ramifié et comportant de 1 à 30 atomes de carbone, ou bien un groupe alkyle cyclique et comportant de 3 à 30 atomes de carbone ;
- R' représente un groupe -CH₂CO₂X lorsque R représente un atome d'hydrogène, tandis que R' représente un atome d'hydrogène lorsque R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium.

Que les dispositifs soient destinés à la décoloration ou à la déformation permanente, les compositions A, A' et B peuvent être des solutions ou se présenter, l'une sous la forme d'une poudre ou d'une crème, et l'autre sous la forme d'une composition aqueuse.

L'invention a, en outre, pour objet l'utilisation d'une composition réductrice, d'un procédé de décoloration ou de déformation permanente, ou d'un dispositif tels que précédemment définis pour la décoloration ou à la déformation permanente de fibres kératiniques humaines et, plus spécialement, de cheveux.

L'utilisation des composés de formule (I) en tant qu'agents complexants dans des compositions pour la décoloration ou la déformation permanente de fibres kératiniques, et notamment de fibres capillaires, présente de nombreux avantages.

En effet, non seulement ces composés manifestent de remarquables propriétés complexantes vis-à-vis des cations métalliques et réduisent, ainsi, considérablement le risque de voir la décoloration ou la déformation permanente s'accompagner d'effets indésirables du type cassure des cheveux ou brûlures du cuir chevelu, mais il s'avère qu'ils sont, de plus, très solubles dans l'eau et stables en milieu aqueux, compatibles avec l'ensemble des composés susceptibles d'entrer dans la constitution de compositions réductrices à visée capillaire, très bien tolérés par la peau, biodégradables, et relativement peu onéreux.

Outre les dispositions qui précèdent, l'invention comprend encore d'autres dispositions qui ressortiront du complément de description qui suit, qui se réfère à des exemples de réalisation de compositions réductrices pour la décoloration d'une part, et pour la déformation permanente d'autre part, de fibres kératiniques.

Il va de soi que ces exemples sont donnés à titre illustratif et en aucun cas limitatif de l'objet de l'invention.

### EXPOSÉ DÉTAILLÉ DE MODES DE RÉALISATION PARTICULIERS

### EXEMPLE 1 : compositions réductrices prêtes à l'emploi pour la décoloration de fibres kératiniques

On a préparé trois compositions réductrices prêtes à l'emploi - respectivement A, B et C - pour la décoloration de fibres kératiniques. Leur composition qualitative et quantitative est présentée dans le tableau I ci-après, dans lequel les quantités des différents constituants sont exprimées en grammes.

**TABLEAU I**

| Constituants | **A** | **B** | **C** |
|---|---|---|---|
| Hydroxyméthane sulfinate de Na | 7 | 7 | --- |
| Acide ascorbique | --- | --- | 10 |
| Sulfite de sodium | --- | --- | 3 |
| Alcool cétylique | 3 | 3 | 3 |
| Laurylsufate de Na | 0,7 | 0,7 | 0,5 |
| Alcool benzylique | 2 | 2 | 2 |
| Propylèneglycol | 10 | 10 | 10 |
| Solution aqueuse à 40% de sel disodique d'acide 2-hydroxyéthyliminodiacétique* | --- | 0,13 | 0,07 |
| Solution aqueuse à 40% de sel trisodique de l'acide méthylglycine diacétique** | 0,15 | --- | --- |
| Solution aqueuse à 85% de H₂PO₄ | q.s.p. pH = 2,7 | | |
| Eau | q.s.p. 100 g | | |

| | | | |
|---|---|---|---|
| * XUS-40855.00 - Société DOW CHEMICAL | | | |
| ** Trilon® M Liquid - Société BASF | | | |

Les compositions A, B et C ont été appliquées, dans un rapport de bain de 10 g de composition pour 1 g de cheveux, sur des cheveux naturellement gris (à 90% de cheveux blancs) et préalablement teintés par la nuance 6.66 de la gamme l'OREAL MAJIROUGE® .

Après 30 minutes de pose, les cheveux ont été rincés abondamment à l'eau, puis traités par une solution aqueuse de H₂O₂ à 3% pendant 2 minutes au terme desquelles les cheveux ont été à nouveau rincés abondamment à l'eau. Les cheveux ont été alors lavés avec un shampooing standard, puis séchés au sèche-cheveux.

On a observé une décoloration puissante et homogène des cheveux traités par les compositions A, B et C. En effet, dans les trois cas, les reflets rouges intenses conférés par la teinture ont pratiquement totalement disparu, laissant apparaître à nouveau les cheveux presque tels qu'ils étaient avant de subir cette teinture.

### EXEMPLE 2 : compositions réductrices prêtes à l'emploi pour la déformation permanente de fibres kératiniques

On a préparé trois compositions réductrices prêtes à l'emploi - respectivement D, E et F - pour la déformation permanente de fibres kératiniques. Leur composition qualitative et quantitative est présentée dans le tableau II ci-après, dans lequel les quantités des différents constituants sont exprimées en grammes.

**TABLEAU II**

| Constituants | **D** | **E** | **F** |
|---|---|---|---|
| Acide thioglycolique | 9,2 | 9,2 | 9,2 |
| Solution aqueuse à 20% de NH₃ | 9,3 | 9,3 | 9,3 |
| Carbonate d'ammonium | 4,5 | 4,5 | 4,5 |
| Solution aqueuse à 30% de cocoylamidopropylbétaïne/monolaurate de glycérol (25:5) | 1,3 | 1,3 | 1,3 |
| Solution aqueuse à 60% d'un polymère cationique de formule W | 1,7 | 1,7 | 1,7 |
| Solution aqueuse à 40% de sel disodique d'acide 2-hydroxyéthyliminodiacétique* | --- | --- | 0,05 |
| Solution aqueuse à 40% de sel trisodique d'acide méthylglycinediacétique** | 0,15 | --- | --- |
| Immunodisuccinate de sodium*** | --- | 1 | --- |
| Eau | q.s.p. 100 g | | |

| | | | |
|---|---|---|---|
| * XUS-40855.00 - Société DOW CHEMICAL | | | |
| ** Trilon® M Liquid - Société BASF | | | |
| *** Iminodisuccinate VP OC sodium salt powder (N-305) - Société BAYER | | | |

Les compositions D, E et F ont été appliquées sur des cheveux humides préalablement enroulés sur des bigoudis de 9 mm de diamètre.

Après 15 minutes de pose, les cheveux ont été rincés abondamment à l'eau, puis traités par une solution aqueuse de H₂O₂ à 8 volumes et de pH 3 pendant 5 minutes au terme desquelles les cheveux ont été rincés une nouvelle fois abondamment à l'eau.

Les bigoudis ont été alors ôtés et les cheveux séchés.

Ces cheveux, qu'ils aient été traités par la composition D, la composition E ou la composition F, ont présenté une belle frisure homogène.

## Revendications

1. Composition réductrice pour la décoloration ou la déformation permanente de fibres kératiniques, comprenant au moins un agent réducteur, **caractérisée en ce qu'**elle comprend au moins un composé répondant à la formule générale (I) suivante :
R-N-(CH(R')CO₂X)₂ (I)
dans laquelle :
• R représente un atome d'hydrogène ou un groupe - CH(CO₂X) - (CH₂)₂CO₂X, -CH₂-CH₂-OH, -CH(CH₃)-CO₂X ou - (CH₂)₂-N (COR") -CH₂-CO₂X ;
• R" représente un groupe alkyle, linéaire ou ramifié et comportant de 1 à 30 atomes de carbone, ou bien un groupe alkyle cyclique et comportant de 3 à 30 atomes de carbone ;
• R' représente un groupe -CH₂CO₂X lorsque R représente un atome d'hydrogène, tandis que R' représente un atome d'hydrogène lorsque R est différent d'un atome d'hydrogène ; et
• X représente un atome d'hydrogène ou un cation monovalent ou divalent issu d'un métal alcalin, d'un métal alcalino-terreux, d'un métal de transition, d'une amine organique, ou un cation ammonium.

2. Composition selon la revendication 1, dans laquelle le cation monovalent ou divalent est choisi dans le groupe constitué par les cations de métaux alcalins, les cations de métaux alcalino-terreux, les cations divalents de métaux de transition, et les cations monovalents issus d'amines organiques ou d'ammonium.

3. Composition selon la revendication 1 ou la revendication 2, **caractérisée en ce que** le ou les composés de formule (I) sont choisis dans le groupe constitué par l'acide méthylglycine diacétique, l'acide 2-hydroxyéthyliminodiacétique, l'acide N-lauroyl-N,N',N'-triacétique éthylènediamine, l'acide iminodisuccinique, l'acide N,N-dicarboxyméthyl L-glutamique, leurs sels de métaux alcalins, leurs sels de métaux alcalino-terreux, leurs sels de métaux de transition, et leurs mélanges.

4. Composition selon l'une quelconque des revendications précédentes, **caractérisée en ce que** le ou les composés de formule (I) sont choisis dans le groupe constitué par l'acide 2-hydroxyéthyliminodiacétique, l'acide méthylglycinediacétique, leurs sels de sodium et leurs mélanges.

5. Composition selon l'une quelconque des revendications précédentes, **caractérisée en ce que** le ou les composés de formule (I) représentent de 0,001 à 10% en poids du poids total de ladite composition.

6. Composition selon l'une quelconque des revendications précédentes, **caractérisée en ce que** le ou les composés de formule (I) représentent de 0,001 à 5% en poids du poids total de ladite composition.

7. Composition selon l'une quelconque des revendications précédentes, **caractérisée en ce que** le ou les agents réducteurs sont choisis dans le groupe constitué par les réductones, leurs sels et leurs esters, les sulfites et les sulfinates.

8. Composition selon l'une quelconque des revendications 1 à 6, **caractérisée en ce que** le ou les agents réducteurs sont choisis dans le groupe constitué par les thiols, leurs sels et leurs esters, les sulfites et les sulfinates.

9. Composition selon la revendication 8, **caractérisée en ce que** le ou les agents réducteurs sont choisis parmi dans le groupe constitué par l'acide thioglycolique, l'acide thiolactique, la cystéamine, la cystéine, leurs sels et leurs esters.

10. Composition selon l'une quelconque des revendications précédentes, **caractérisée en ce que** le ou les agents réducteurs représentent de 0,1 à 30% en poids du poids total de ladite composition.

11. Composition selon l'une quelconque des revendications précédentes, **caractérisée en ce que** le ou les agents réducteurs représentent de 0,5 à 20% en poids du poids total de ladite composition.

12. Composition selon l'une quelconque des revendications précédentes, **caractérisée en ce qu'**elle comprend, de plus, un ou plusieurs polymères conditionneurs cationiques ou amphotères, dans des proportions de 0,01 à 10% en poids et, de préférence, de 0,05 à 5% en poids du poids total de ladite composition.

13. Composition selon l'une quelconque des revendications précédentes, **caractérisé en ce qu'**elle comprend, de plus, un ou plusieurs polymères amphiphiles non ioniques, anioniques, cationiques ou amphotères, comportant une chaîne hydrophobe, dans des proportions de 0,05 à 20% en poids et, de préférence, de 0,1 à 10% en poids du poids total de ladite composition.

14. Composition selon l'une quelconque des revendications précédentes, **caractérisée en ce qu'**elle comprend, de plus, un ou plusieurs agents tensioactifs, dans des proportions de 0,01 à 40% en poids et, de préférence, de 0,1 à 30% en poids du poids total de ladite composition.

15. Composition selon l'une quelconque des revendications précédentes, **caractérisée en ce qu'**elle comprend, de plus, un ou plusieurs agents d'ajustement de la rhéologie différents des polymères amphiphiles non ioniques, anioniques, cationiques ou amphotères, comportant une chaîne hydrophobe, dans des proportions de 0,05 à 20% en poids et, de préférence, de 0,1 à 10% en poids du poids total de ladite composition.

16. Composition selon l'une quelconque des revendications précédentes, **caractérisée en ce qu'**elle comprend, de plus, un ou plusieurs agents acidifiants ou alcalinisants, dans des proportions de 0,01 à 30% en poids du poids total de ladite composition.

17. Composition selon l'une quelconque des revendications précédentes, **caractérisée en ce qu'**elle comprend, de plus, un ou plusieurs solvants choisis dans le groupe constitué par l'eau et les mélanges composés d'eau et d'un ou plusieurs solvants organiques acceptables sur le plan cosmétique, ce ou ces solvants représentant de 0,5 à 20% en poids et, de préférence, de 2 à 10% en poids du poids total de ladite composition.

18. Composition selon l'une quelconque des revendications précédentes, **caractérisée en ce qu'**elle comprend, de plus, un ou plusieurs adjuvants choisis dans le groupe constitué par les charges minérales ou organiques, les liants, les lubrifiants, les agents antimousse, les silicones, les agents colorants, les agents matifiants, les conservateurs et les parfums.

19. Procédé de décoloration ou de déformation permanente de fibres kératiniques, comprenant les étapes consistant à :
a) appliquer sur les fibres kératiniques une composition réductrice selon l'une quelconque des revendications 1 à 18 ;
b) laisser reposer la composition réductrice sur les fibres kératiniques pendant un temps suffisant pour obtenir la décoloration ou la déformation recherchée ;
c) rincer les fibres kératiniques pour en éliminer la composition réductrice ;
d) laver les fibres kératiniques une ou plusieurs fois, les rincer après chaque lavage et, éventuellement, les sécher ;
ledit procédé comprenant de plus, entre les étapes c) et d), dans le cas d'une déformation permanente, les étapes consistant à : i) appliquer sur les fibres kératiniques une composition oxydante ; ii) laisser reposer la composition oxydante sur les fibres kératiniques pendant un temps suffisant pour obtenir la fixation de la déformation recherchée ; et iii) rincer les fibres kératiniques à l'eau pour en éliminer la composition oxydante.

20. Dispositif ou "kit" pour la décoloration de fibres kératiniques, comprenant au moins deux compositions A et B destinées à être mélangées ensemble pour obtenir une composition réductrice prête à l'emploi, **caractérisé en ce que** l'une au moins des compositions A et B contient un ou plusieurs agents réducteurs, et l'une au moins des compositions A et B contient un ou plusieurs composés répondant à la formule générale (I) suivante :
R-N-(CH(R')CO₂X)₂ (I)
dans laquelle :
• R représente un atome d'hydrogène ou un groupe - CH(CO₂X) - (CH₂)₂CO₂X, -CH₂-CH₂-OH, -CH (CH₃) -CO₂X ou - (CH₂)₂-N (COR") -CH₂-CO₂X ;
• R" représente un groupe alkyle, linéaire ou ramifié et comportant de 1 à 30 atomes de carbone, ou bien un groupe alkyle cyclique et comportant de 3 à 30 atomes de carbone ;
• R' représente un groupe -CH₂CO₂X lorsque R représente un atome d'hydrogène, tandis que R' représente un atome d'hydrogène lorsque R est différent d'un atome d'hydrogène ; et
• X représente un atome d'hydrogène ou un cation monovalent ou divalent issu d'un métal alcalin, d'un métal alcalino-terreux, d'un métal de transition, d'une amine organique, ou un cation ammonium.

21. Dispositif ou "kit" pour la déformation permanente de fibres kératiniques, comprenant, d'une part, soit une composition A, soit au moins deux compositions A' et B' destinées à être mélangées ensemble pour obtenir une composition réductrice prête à l'emploi, et, d'autre part, une composition oxydante C prête à l'emploi ou au moins deux compositions D et E destinées à être mélangées ensemble pour obtenir une composition oxydante prête à l'emploi, **caractérisé en ce que** soit la composition A, soit l'une au moins des compositions A' et B' contient un ou plusieurs agents réducteurs, et soit la composition A, soit l'une au moins des compositions A' et B' contient au moins un ou plusieurs composés répondant à la formule générale (I) suivante :
R-N-(CH(R')CO₂X)₂ (I)
dans laquelle :
• R représente un atome d'hydrogène ou un groupe -CH(CO₂X) - (CH₂)₂CO₂X, -CH₂-CH₂-OH, -CH (CH₃) -CO₂X ou - (CH₂)₂-N (COR") -CH₂-CO₂X ;
• R" représente un groupe alkyle, linéaire ou ramifié et comportant de 1 à 30 atomes de carbone, ou bien un groupe alkyle cyclique et comportant de 3 à 30 atomes de carbone ;
• R' représente un groupe -CH₂CO₂X lorsque R représente un atome d'hydrogène, tandis que R' représente un atome d'hydrogène lorsque R est différent d'un atome d'hydrogène ; et
• X représente un atome d'hydrogène ou un cation monovalent ou divalent issu d'un métal alcalin, d'un métal alcalino-terreux, d'un métal de transition, d'une amine organique, ou un cation ammonium.

22. Utilisation d'une composition selon l'une quelconque des revendications 1 à 18, ou d'un procédé selon la revendication 19, ou d'un dispositif selon la revendication 20 ou la revendication 21, pour la décoloration ou à la déformation permanente de fibres kératiniques humaines et, plus spécialement, de cheveux.
